**Europäisches Patentamt**

**European Patent Office**

**Office européen des brevets**

(19)

(11) Veröffentlichungsnummer:

# 0 031 790
## A1

(12) ## EUROPÄISCHE PATENTANMELDUNG

(21) Anmeldenummer: 80730069.4

(51) Int. Cl.³: **A 61 L 11/00**, B 65 F 7/00

(22) Anmeldetag: 30.10.80

(30) Priorität: 10.12.79 DE 2950028
14.10.80 DE 3039173

(43) Veröffentlichungstag der Anmeldung: 08.07.81
**Patentblatt 81/27**

(84) Benannte Vertragsstaaten: **AT BE CH FR GB IT LI LU NL SE**

(71) Anmelder: **Drauschke, Alfred, Heidestrasse 19, D-1000 Berlin 28 (DE)**

(72) Erfinder: **Drauschke, Alfred, Heidestrasse 19, D-1000 Berlin 28 (DE)**

(74) Vertreter: **Meinig, Karl-Heinz, PATENTANWÄLTE-PFENNING-MAAS-MEINIG-SPOTT Kurfürstendamm 170, D-1000 Berlin 15 (DE)**

(54) **Verfahren zum Sterilisieren und zur Entsorgung infektiöser Materialien, wie Krankenhausabfälle und anderes mehr und Vorrichtung zur Durchführung des Verfahrens.**

(57) Im Vorliegenden wird ein Verfahren beschrieben, welches insbesondere der Sterilisierung und Entsorgung von infektiösen Materialien dient, wie sie beispielsweise im Zusammenhang mit verschiedenartigsten Krankenhausabfällen anfallen, aber auch in Form von infektiös befallenen Lebensmitteln oder dergleichen Güter vorliegen können. Das Verfahren beinhaltet dabei eine Vakuum-Dampf-Sterilisation, bei der die Materialien, soweit es sich um nasse Abfälle, beispielsweise verdorbenes Blut, Rückstände aus dem OP-Bereich von Krankenhäusern, aus der Pathologie handelt, eine Homogenisierung der sterilisierten Masse und eine Trockenaufbereitung, während auf die Homogenisierung bei trockenen infektiösen Massen in aller Regel verzichtet werden kann.

Für letztere Materialien ist es besonders vorteilhaft, zur Durchführung verfahrensweise einen Sterilisator einzusetzen, der, auf einem Kraftfahrzeug oder dergleichen verfahrbaren Gestell montiert, eine Zusammenfassung von an unterschiedlichen Orten anfallenden infektiösen Materialien ermöglicht, die dann unmittelbar in diesem als Transportbehälter diesenden Sterilisator an eine geeignete Dampf-Vakuum- und Unterdruckquelle angeschlossen sterilisierbar sind.

ACTORUM AG

Die vorliegende Erfindung bezieht sich auf ein Verfahren zur Entsorgung und Sterilisation infektiöser Materialien nach dem Gattungsteil der Patentansprüche 1 und 2 sowie auf eine Vorrichtung zur Durchführung des Verfahrens.

An die Sterilisation von infektiös kontaminierten Materialien werden seitens des Gesetzgebers im Interesse des Allgemeinwohls und der Gesunderhaltung der Bevölkerung hohe Anforderungen gestellt, so daß in jedem Fall sichergestellt ist, daß mit Viren, Bakterien oder dergleichen Krankheitserregern befallenes Gut aber auch in Infektionsstationen von Krankenhäusern verwendetes Material nicht vor ausreichender Sterilisation unkontrollierbar in die Außenwelt gelangt. So ist es beispielsweise bisher üblich, Verbandsmaterial, Einwegspritzen, Tupfer, Papierhandtücher, Papierwäsche und dergleichen in Krankenhäusern anfallendes Material in besonderen Behältern zu sammeln und innerhalb des Krankenhauses in einer Sterilisationsabteilung keimfrei zu machen oder unter Einhaltung besonderer Sicherheitsmaßnahmen zu verbrennen.

Die Vorschriften des Abfallbeseitigungsgesetzes besagen unter anderem, daß Körperteile und Organabfälle aus dem Bereich der Pathologie, Chirurgie, Gynäkologie und Geburtenhilfe, der Blutbank und

- 2 -

dergleichen im allgemeinen verbrannt werden müssen, während Speise- und Küchenabfälle, insbesondere aus dem infektiösen Bereich von Krankenhäusern, einer besonderen Sterilisationsbehandlung bedürfen.

Bisher wird versucht, dem Abfallbeseitigungsgesetz dadurch gerecht zu werden, daß zumindest größere Krankenhäuser mit Müllsterilisationsanlagen, teilweise auch Verbrennungsanlagen ausgestattet sind, in denen diegenannten Abfälle zusammengefaßt werden, um in einem Autoklaven unschädlich gemacht zu werden. Diese Anlagen sind jedoch nicht nur außerordentlich personalintensiv, sondern sie bieten auch eine erhöhte Gefahr der Kontamination der unmittelbaren und weiteren Umgebung und schließlich auch eine Umweltbelästigung durch den unangenehmen Geruchsanfall bei der Verbrennung der Abfälle durch ein Stützfeuer. Das Stützfeuer ist nur mit einem erheblichen Energieaufwand realisierbar, wobei bei den in der Praxis meist diskontinuierlichen Anlagen hinzukommt, daß eine Verbrennung von der Müllabfuhr nichtzuführbaren Krankenhausabfällen erst dann erfolgen kann, wenn die Anlage zuvor ausreichend aufgeheizt und damit mit Wärmeenergie beaufschlagt worden ist.

Die manuelle Entladung des Autoklaven oder wenigstens die manuelle Steuerung dieser Vorgänge bürgt eine Vielzahl von Fehlermöglichkeiten in sich, und die Transportmittel von und zu der Müllsterilisations-

- 3 -

anlage müssen zusätzlich ständig gewartet und für sich soweit als möglich steril gehalten werden. Hierfür sind zusätzliche Wasch- und Desinfektionsanlagen mit den erforderlichen Investitionskosten unumgänglich. Die Störanfälligkeit der Verbrennungsanlage unter Verwendung eines Stützfeuers für teigige oder nasse Krankenhausabfälle liegt durch den benötigten hohen Temperaturaufwand auf der Hand, was die ohnehin vorhandenen Probleme bei solchen Anlagen zusätzlich nachteilig kompliziert. Die geforderte Aufteilung der Anlage in eine sogenannte reine und eine unreine Seite läßt sich in der Praxis, wenn überhaupt, meist nur schwer realisieren. Hierdurch entstehen den Krankenhäusern nicht unerhebliche Kosten. Die für die Sterilisation den Vorschriften entsprechenden Anlagen müssen für jedes Krankenhaus extra errichtet oder bereits vorhandene ständig kontrolliert und gegebenenfalls erheblich verbessert oder durch neue den erhöhten Anforderungen genügende ersetzt werden.

Hier setzt die vorliegende Erfindung ein, der die Aufgabe zugrunde liegt, Verfahren der im Gattungsteil der Verfahrensansprüche genannten Art dahingehend zu verbessern, daß bei Wegfall von Umweltbelästigungen und bei optimaler Lösung des Energieproblems für die Entsorgung eine einwandfrei sterilisierende Aufarbeitung von infektiös befallenen Materialien oder Abfällen sichergestellt wird

- 4 -

und darüber hinaus insbesondere auch für trockne,
voluminöse Materialien eine Zentralisierung der
Sterilisation möglich wird.

Die Lösung dieser Aufgabe wird erfindungsgemäß
durch die Kombination der im Kennzeichen des
Anspruchs 1 angegebenen Verfahrensschritte sowie
durch ein nebengeordnetes Verfahren gemäß
Anspruch 2 erreicht, und durch eine Vorrichtung
zur Durchführung des Verfahrens entsprechend
den Unteransprüchen.

Dadurch, daß die Krankenhausabfälle, so wie sie in
den einzelnen Bereichen anfallen, wahllos in
Einwegtransportbehältern zusammengefaßt werden,
die dann dauerhaft verschließbar sind, um einer
zentralen Entsorgungsanlage zugeführt zu werden,
deren Kernstück eine an sich bekannte Vakuum-Dampf-
Sterilisationsvorrichtung ist, und das homogenisierte Produkt einer Trocknung unterworfen wird,
ergeben sich folgende Vorteile:

Die bisherigen Einzelanlagen für annähernd jedes
größere Krankenhaus mit ihren Unzulänglichkeiten
innerhalb einer Stadt entfallen und können durch
eine Anlage, die vorzugsweise in unmittelbarer
Nähe eines Müllverbrennungskraftwerkes installierbar ist, zusammengefaßt werden, was eine erhebliche
Verbesserung der Gesamtentsorgung bedeutet.

Die bisher unumgängliche personalintensive Beschickung und Handhabung der bekannten Müllsterilisationsanlagen wie auch der Abfall Verbrennungseinrichtungen entfällt nahezu gänzlich. Die durch das bei Verbrennungsanlagen benutzte Stützfeuer benötigte Energie und die durch die auftretenden erheblichen Temperaturgradienten innerhalb der Verbrennungsanlage unvermeidlichen Defekte und Reparaturarbeiten sind bei dem hier vorliegenden Verfahren gleichfalls kein Problem mehr. Kontaminationsprobleme der bisher bekannten stets wiederverwendeten Container, ihrer Transporteinrichtungen und sonstigen Zubehöres treten nicht mehr auf und entsprechend kann auch hierfür verwendete Wasch- und Desinfektionsanlagen verzichtet werden. Die Aufteilung in eine sogenannte unreine und eine reine Seite der Sterilisationsanlage läßt sich bei der vorteilhaften Verwendung eines Trommelsterilisators einfach bewerkstelligen und bietet sich geradezu an. Damit können sich bei der erfindungsgemäßen Verfahrensweise sowohl die Investitions- als auch die laufenden Betriebskosten erheblich senken lassen, wobei nicht zuletzt der energetische Gesichtspunkt und der des Wegfalls der Umweltbelastung von ganz erheblicher Bedeutung sind. Auch bisher stets nicht ganz von der Hand zu weisende hygienische Gesichtspunkte bei der Verbrennung des hier interessierenden Krankenhausabfalls, etwa durch nur unzureichende Verbrennung von Organteilen aus der Pathologie, sind mit Sicherheit ausgeschaltet.

Die Zusammenfassung der Entsorgung in einer einzigen Anlage, beispielsweise für eine Stadt für eine Mehrzahl von Krankenhäusern, macht zwar ein Umladen von Einwegcontainern und deren Transport zu der Zentralentsorgungsanlage erforderlich, es muß hier jedoch betont werden, daß auch bisher für die Einzelanlagen ein Umladen der im Recycling-System verwendeten Container, und zwar dort von Hand, unumgänglich war, wobei Verletzungsgefahren des Transportpersonals nicht auszuschließen waren, was mit den nunmehr zum Einsatz kommenden Einwegbehältern aus Kunststoff, die in toto zusammen mit dem Abfall in einen Trommelsterilisator oder dergleichen eingebracht werden, nicht mehr gegeben ist.

Das Gesamtverfahren arbeitet nach dem Prinzip höchster Funktionssicherheit und geringster Reparaturanfälligkeit. Die für die Betreibung des Dampfsterilisators erforderliche Energie wird beispielsweise aus dem Abfalldampf einer Müllverbrennungsanlage eines benachbarten Kraftwerkes entnommen, dem wiederum der Rückstand beziehungsweise das durch das Verfahren erhaltene Produkt, welches einen hohen Heizwert hat, zugeführt werden kann, wobei letztlich eine nicht unerhebliche Überschußwärme erzeugt wird, da für 1 kg Organabfälle zur Sterilisation und Trocknung ca. 1 kg Dampf benötigt werden, was bei Berücksichtigung der Aufheiz- und Abstrahlungsverluste ca. 600 kcal. entspricht, und wobei sich ein

0031790

- 7 -

brennbarer Rückstand von 0,3 kg mit einem Heizwert von 5800 kcal. ergibt, also ein Energiegewinn von insgesamt 1740 kcal. Demgegenüber sind für die Verbrennung eines Kilogramms Organabfälle nach der bisherigen Methode unter Verwendung eines Stützfeuers in einem Verbrennungsofen ca. 0,3 bis 0,5 kg Heizöl erforderlich, was einen Energieaufwand von ca. 3000 bis 5000 kcal. entspricht, wobei das Endprodukt Asche also ein nicht weiterverwertbares Material ist.

Im allgemeinen wird bei der vorliegenden Verfahrensweise in der ersten bevorzugten Ausführungsform folgendermaßen vorgegangen:

Krankenhausabfälle, wie Organreste aus dem OP-Bereich, der Pathologie oder nasse beziehungsweise trockene infektiöse Abfälle, aber auch in der Urologie, Gynäkologie, Geburtenhilfe und im Laborbereich anfallende nicht der normalen Müllabfuhr zuführbare Abfälle, werden wahllos zusammen mit solchen Abfällen, wie verdorbenem Blut, Restmedikamenten und ihrer Verpackung, Einwegspritzen, Leerbehältern aus Glas, Kunststoff, verbrauchten Metallinstrumenten und dergleichen mehr, in transportsicheren und praktisch unzerstörbaren sicherverschließbaren Einwegbehältern zusammengefaßt, und diese Einwegbehälter in Tiefkühlanlagen gegebenenfalls so lange aufbewahrt, bis ein Sammeltransport zur zentralen Entsorgungsstation angezeigt ist. Dieser Sammeltransport in

- 8 -

Spezialkühlwagen ist für die Umwelt ungefährlich,
da er so ausgelegt ist, daß auch bei nicht auszuschließenden Unfällen die Einwegbehälter umschlossen
auf dem Transprotwagen verbleiben und möglicherweise zwar deformierbar, jedoch nicht zerstörbar sind.

In der zentralen Entsorgungsanlage werden die
Einwegbehälter ohne geöffnet zu werden zusammen
mit ihrem Inhalt unmittelbar in einen Vakuum-Dampf-
Sterilisator, der vorzugsweise in Form eines
Trommelsterilisators ausgebildet ist, geworfen
und hier einem Dampfdruck von 4 atü sowie einer
Mindeststerilisationstemperatur von 134$^{\circ}$C ausgesetzt. Der Trommelsterilisator ist so ausgelegt,
daß er gleichzeitig eine mechanische Zerkleinerung
und Homogenisierung der durch die erhöhte Druck-
und Temperaturbeaufschlagung nicht zerstörbaren
Anteile, wie etwa des Einwegbehälters, Medikamtenflaschen, Einwegspritzen und dergleichen mehr,
in an sich bekannter Weise sicherstellt. Das durch
dieses Sterilisationsverfahren entstehende Homogenisat wird dann entweder in einem nachgeschalteten Trockner oder unmittelbar in dem Trommelsterilisator auf einen Feuchteanteil von nur
noch ca. 10% Wassergehalt heruntergetrocknet, so
daß sich ein mehlartiges oder körniges brennbares
Produkt von sehr hohem Heizwert, der etwa demjenigen der Steinkohle gleichkommt, entsteht, das
dann der sogenannten reinen Seite der Sterilisationsvorrichtung entnommen werden kann, um entweder

- 9 -

einem Speicher oder direkt einem Energieerzeuger, wie einem Müllverbrennungskraftwerk oder dergleichen, zugeführt zu werden.

Wesentlich für die zweite bevorzugte Ausführungsform des vorliegenden Verfahrens, das insbesondere für trockne, voluminöse Materialien vorteilhaft ist, ist,daß das die infektiösen Materialien transportierende Fahrzeug gewissermaßen der Sterilisator selbst ist, beziehungsweise als Lastkraftwagen ausgebildet den Sterilisator in Form eines Druckbehälters aufnimmt. Ein solches Fahrzeug kann problemlos an jede Sammelstation für infektiöse Abfälle aber auch an ein Lager, in dem sich verdorbene oder befallene Lebensmittel und dergleichen befinden, heranfahren, das Material nehmen und dann hermetisch gegen die Außenwelt verschlossen an eine beliebige Dampfquelle geeigneter Art herangefahren werden, woraufhin dann der Sterilisationsvorgang in dem Druckbehälter des Fahrzeugs unmittelbar durchgeführt wird, ohne daß ein nochmaliges Umladen in einen stationär installierten Sterilisator erforderlich wird. Bei der üblich gegebenen Kapazität infektiös anfallender Materialien innerhalb eines kommunalen Bevölkerungsgebietes entsorgt ein solches Spezialfahrzeug eine Vielzahl von Krankenhäusern oder anderen Stationen problemlos.

0031790

Die Dampf- und Unterdruckquelle, zu der das Fahrzeug für den Vorgang der Dampf-Vakuum-Sterilisation zu fahren ist, kann hierbei beliebig gewählt werden, wobei die jeweils örtlich gegebenen speziellen Verhältnisse, wie das Vorhandensein eines Müllverbrennungskraftwerks, eines Ferndampfheizwerks, die jeweils wirtschaftlichste Lösung offen lassen. Das Sterilisatorfahrzeug kann auf diese Weise innerhalb eines abgeschlossenen Kommunalbereichs eine oder mehrere Kuppelstationen anfahren, um das Sterilisationsverfahren durchzuführen; es ist jedoch auch denkbar, daß der Dampf mittels eines entsprechenden Dampferzeugers und der Unterdruck mittels einer Vakuumpumpe direkt im Fahrzeug erzeugt werden. Dort, wo in kommunalen Bereichen zentrale Entsorgungsstationen und/oder Müllverbrennungsanlagen vorhanden sind, bietet sich eine solche Anlage als Kuppelstation für die Übernahme von Fremddampf in den Fahrzeugsterilisator und die Abgabe des sterilisierten Materials in die Müllverbrennungsanlage selbstverständlich als besonders vorteilhafte Lösung an.

Die dem Sterilisator entnommenen keimfreien Materialien können je nach deren Natur und Gegebenheit wahlweise verbrannt, deponiert oder auch anderweitig verwendet werden.

- il -

Während des Beladevorgangs des Sterilisator
ist in jedem Fall dafür zu sorgen, daß
innerhalb desselben ein Unterdruck vorhanden
ist, so daß kein infektiöses Gut in die
Umwelt gelangen kann.

– 12 –

ANSPRÜCHE

1.  Verfahren zur Sterilisierung und Entsorgung von der Müllabfuhr nichtzuführbaren Krankenhausabfällen, wie Organabfällen aus dem OP-Bereich, der Pathologie, nassen und/oder trockenen infektiösen Abfällen aber auch befallener Nahrungsmittel und dergleichen mehr, g e k e n n z e i c h n e t   d u r c h die Kombination der folgenden Verfahrensschritte:

1. die Abfälle werden in Einwegbehältern wahllos zusammengefaßt und verschlossen;

2. die Einwegbehälter werden einer zentralen Entsorgungsanlage zugeführt; und

3. innerhalb der Entsorgungsanlage in toto einem Vakuum-Dampf-Sterilisationsverfahren unterworfen und hierbei die unverkochbaren Teile mechanisch homogenisiert;

4. nachfolgend erfolgt eine Trocknung bis auf einen Feuchteanteil von ca. 10% Wasser; und

5. das so erhaltene, leicht brennbare Produkt wird der Sterilisationsvorrichtung an einer der Eingabeseite der Einwegbehälter entfernt liegenden und hiervon getrennten reinen Seite entnommen.

2. Verfahren zur Sterilisierung und Entsorgung infektiöser Materialien, wie entsprechende Krankenhausabfälle, infektiös befallene Nahrungsmittel und dergleichen mehr, d a d u r c h   g e k e n n z e i c h n e t , daß

1. die vorzugsweise trockenen Materialien in Einwegbehältern oder dergleichen Aufnahmegefäßen wahllos zusammengefaßt und verschlossen werden;

2. die Einwegbehälter einem Sterilisator zugeführt werden;

3. innerhalb dieses Sterilisators unter wechselnder Anwendung von Wasserdampf und Unterdruck ein Vakuum-Dampf-Sterilsationsverfahren durchgeführt wird, wobei das in den Behälter zusammengefaßte Material in einen auf einem Fahrzeug montierten Sterilisator behandelt wird; und

4. der Sterilisator für den Sterilisationsvorgang unmittelbar an diese zu einer Wasserdampf- und Unterdruckstation gefahren wird; und

5. nach Durchführung des Vakuum-Dampf-Sterilisationsverfahrens das keimfreie Material entnommen wird.

- 14 -

3. Vorrichtung zur Durchführung des Verfahrens
nach Anspruch 2, dadurch gekennzeichnet,
daß der Sterilisator ein auf einem Kraftfahrzeug montierter Druckbehälter mit einem dicht
verschließbaren Kupplungsanschluß für die
Dampf- und Unterdruckquelle ist.

4. Vorrichtung zur Durchführung des Verfahrens
nach Anspruch 1 und 2, dadurch gekennzeichnet,
daß das keimfreie Material aus dem Sterilisator
entnommen unterschiedlichen Verwendungszwecken
zuführbar ist.

**Europäisches Patentamt**

# EUROPÄISCHER RECHERCHENBERICHT

**0031790**
Nummer der Anmeldung

**EP 80 73 0069**

| Kategorie | EINSCHLÄGIGE DOKUMENTE | betrifft Anspruch | KLASSIFIKATION DER ANMELDUNG (Int. Cl.³) |
|---|---|---|---|
| | Kennzeichnung des Dokuments mit Angabe, soweit erforderlich, der maßgeblichen Teile | | |
| | US - A - 2 708 074 (J.K. HOSKINS et al.) * Spalte 6, Zeilen 14-46 * -- | 1 | A 61 L 11/00 B 65 F 7/00 |
| | US - A - 2 731 208 (T.G. DODD) * Spalte 5, Zeilen 25-41 * -- | 1 | |
| | DE - A - 2 621 750 (O. ANNA) * Patentanspruch 3 * -- | 1 | |
| A | DE - A - 2 455 791 (FISCHINGER & MAYER) ----- | 3 | |

**RECHERCHIERTE SACHGEBIETE (Int. Cl.³)**

A 61 L 11/00
A 61 L 2/06
B 65 F 7/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 24.03.1981 | PELTRE |

EPA form 1503.1  06.78